# EUROPEAN PATENT APPLICATION

(11) **EP 2 865 749 A1**
(43) Date of publication of application: **29.04.2015**
(21) Application number: 13810037.5
(22) Date of filing: 23.05.2013
(51) Int. Cl.: C12N 5/0775, C12N 5/02

(54) **HIGH-CONCENTRATION STEM CELL PRODUCTION METHOD**

(30) Priority: 26.06.2012 KR 20120068796
(71) Applicant: Ra, Jeong Chan, Suwon-si, Gyeonggi-do 440-300 (KR); K-Stemcell Co., Ltd., Seoul 150-870 (KR)
(72) Inventor: KANG, Sung Keun, Seoul 151-770 (KR); JO, Jung Youn, Seoul 138-859 (KR); RA, Jeong Chan, Suwon-si Gyeonggi-do 440-300 (KR)
(74) Representative: Hiebl, Inge Elisabeth
(86) International application number: PCT/KR2013/004517
(87) International publication number: WO 2014/003319

(57) **Abstract**

The present invention relates to a method for preparing stem cells in high concentration. The present invention makes it possible to grow stem cells in an amount sufficient to be clinically usable in a short time, and makes it possible to relatively efficiently enhance the ability of administered stem cells to efficaciously reach target tissue and exhibit an action in a stable fashion and can therefore dramatically increase the efficacy of cell therapy using stem cells.

## Description

### TECHNICAL FIELD

The present invention relates to a method for preparing a high concentration of stem cells, and more particularly to a method for growing stem cells in high yield sufficient for clinical application.

### BACKGROUND ART

Stem cells refer to cells having not only self-replicating ability but also the ability to differentiate into at least two types of cells, and can be divided into totipotent stem cells, pluripotent stem cells, and multipotent stem cells. Totipotent stem cells are cells having totipotent properties capable of developing into one perfect individual, and these properties are possessed by cells up to the 8-cell stage after the fertilization of an oocyte and a sperm. When these cells are isolated and transplanted into the uterus, they can develop into one perfect individual. Pluripotent stem cells, which are cells capable of developing into various cells and tissues derived from the ectodermal, mesodermal and endodermal layers, are derived from an inner cell mass located inside of blastocysts generated 4-5 days after fertilization. These cells are called "embryonic stem cells" and can differentiate into various other tissue cells but not form new living organisms. Multipotent stem cells, which are stem cells capable of differentiating into only cells specific to tissues and organs containing these cells, are involved not only in the growth and development of various tissues and organs in the fetal, neonatal and adult periods but also in the maintenance of homeostasis of adult tissue and the function of inducing regeneration upon tissue damage. Tissue-specific multipotent cells are collectively called "adult stem cells".

Adult stem cells are obtained by taking cells from various human organs and developing the cells into stem cells and are characterized in that they differentiate into only specific tissues. However, recently, experiments for differentiating adult stem cells into various tissues, including liver cells, were dramatically successful, which comes into spotlight. In particular, efforts have been made in the field of regenerative medicine for regenerating biological tissues and organs and recovering their functions that were lost due to illness or accident and the like by using cells. Methods which are frequently used in this field of regenerative medicine comprise the steps of: collecting stem cells, blood-derived mononuclear cells or marrow-derived mononuclear cells from a patient; inducing the proliferation and/or differentiation of the cells by tube culture; and introducing the selected undifferentiated (stem cells and/or progenitor cells) and/or differentiated cells into the patient's body by transplantation. Accordingly, existing classical methods for treating diseases by medication or surgery are expected to be replaced with cell/tissue replacement therapy which replaces a damage cell, tissue or organ with healthy one, and thus the utility of stem cells will further increase.

Thus, the various functions of stem cells are currently being studied. Particularly, since cell therapy technologies using mesenchymal stem cells started to receive attention, technologies for improving mesenchymal stem cells isolated from a human body so as to be suitable for therapeutic purposes have been developed (WO 2006/019357, Korean Patent No. 0795708, and Korean Patent No. 0818214).

However, a technology related to a method for proliferating the isolated stem cells in an amount sufficient for clinical application has not yet been sufficiently studied.

Accordingly, the present inventors have made many effort to prepare stem cells in large amounts, and have found that, when stem cells are cultured in a medium containing a basal medium and at least two components selected from the group consisting of N-acetyl-L-cysteine (NAC), ascorbic acid, insulin or insulin-like factor, hydrocortisone, basic fibroblast growth factor (bFGF), EGF (epidermal growth factor), and antioxidant, they can be proliferated at a high concentration in a short period of time, thereby completing the present invention.

The above information disclosed in this Background section is only for enhancement of understanding of the background of the present invention, and therefore it may contain information that does not form the prior art that is already known to a person of ordinary skill in the art.

### DISCLOSURE OF INVENTION

### TECHNICAL PROBLEM

It is an object of the present invention to provide a method for preparing stem cells at a high concentration in a short period of time.

### TECHNICAL SOLUSION

To achieve the above objects, the present invention provides a method for preparing a high concentration of stem cells, the method comprising of culturing stem cells in a medium containing a basal medium; and at least two components selected from the group consisting of N-acetyl-L-cysteine (NAC), ascorbic acid, insulin or insulin-like factor, hydrocortisone, dexamethasone, bFGF (basic fibroblast growth factor), heparan sulfate, 2-mercaptoethanol, EGF (epidermal growth factor), and antioxidant.

Other features and embodiments of the present invention will be more apparent from the following detailed descriptions and the appended claims.

### BRIEF DESCRIPTION OF THE DRAWINGS

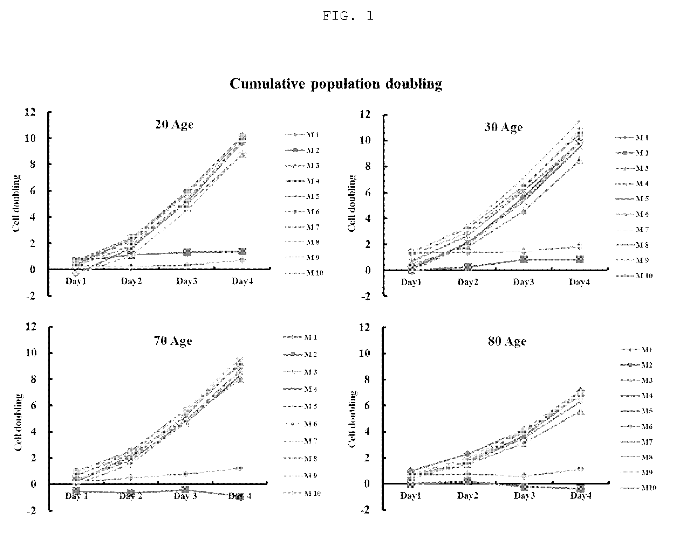

FIG. 1 is a graphic diagram showing a cell population doubling level during the culture of adipose-derived stem cells obtained from adult males aged 20, 30, 70 and 80 years in each of media for 4 days.

### BEST MODE FOR CARRYING OUT THE INVENTION

Unless defined otherwise, all technical and scientific terms used herein have the same meaning as commonly understood by one of ordinary skill in the art to which the invention pertains. Generally, the nomenclature used herein and the experiment methods are those well known and commonly employed in the art.

As used herein, the term "stem cells" refer to cells having not only self-replicating ability but also the ability to differentiate into at least two types of cells. "Adult stem cells" refer to stem cells that appear either in the stage in which each organ of an embryo is formed after the developmental process or in the adult stage.

As used herein, the term "mesenchymal stem cells" refers to undifferentiated stem cells that are isolated from human or mammalian tissue and may be derived from various tissues. Particularly, the mesenchymal stem cells may be umbilical cord-derived mesenchymal stem cells, umbilical cord blood-derived mesenchymal stem cells, bone marrow-derived mesenchymal stem cells, adipose-derived mesenchymal stem cells, muscle-derived mesenchymal stem cells, nerve-derived mesenchymal stem cells, skin-derived mesenchymal stem cells, amnion-derived mesenchymal stem cells, or placenta-derived mesenchymal stem cells. Technology for isolating stem cells from each tissue is already known in the art.

As used herein, "adipose tissue-derived mesenchymal stem cells" are undifferentiated adult stem cells isolated from adipose tissue and are also referred to herein as "adipose-derived adult stem cells", "adipose stem cells", or "adipose-derived stem cells". These cells can be obtained according to any conventional method known in the art. A method for isolating adipose tissue-derived mesenchymal stem cells may be, for example, as follows. That is, adipose-derived mesenchymal stem cells can be isolated by culturing an adipose-containing suspension (in physiological saline) obtained by liposuction, and then either collecting a stem cell layer, attached to a culture container such as a flask, by trypsin treatment, or directly collecting those suspended in a small amount of physiological saline by rubbing with a scraper.

As used herein, the terms "preparation of a high concentration of stem cells" means that the number of stem cells proliferates in a high yield. As used herein, the terms "high concentration of stem cells" is also referred to as "large number of stem cells". In an example of the present invention, it was confirmed that when stem cells isolated tissue are cultured in a medium according to the present invention, the number of stem cells obtained is significantly increased. Preferably, when it was confirmed that when subcultured 3-5 times, the stem cells can be obtained at a concentration of 1 × 10⁷ - 5 × 10⁶ cells/ml.

As used herein, the term "subculture" means that a portion of cells is periodically passaged by transferring them to a new culture vessel and by replacing the culture medium with a fresh culture for the long-term culture of healthy cells. As the number of cells increases in the limited space of the culture vessel, the cells die naturally due to nutrient depletion or waste accumulation in a predetermined time. Thus, subculture is used to increase the number of healthy cells. Typically, 1 passage means culture by one replacement of medium (culture vessel) or one isolation of cell population. Any subculture method known in the art may be used without any limitation, but a mechanical or enzymatic isolation method may be preferably performed.

As used herein, the term "mechanical isolation" means that cell aggregates are isolated physically or mechanically. Any mechanical isolation method known in the art may be used without any limitation, but cells may be preferably isolated by using a blade, a tissue chopper, a needle, pipetting, an EBD (embryoid body divider) or a scrapper. According to a preferred embodiment of the present invention, it was confirmed that large-scale proliferation of stem cells can be achieved by subculturing them using the blade or the tissue chopper.

As used herein, the term "enzymatic isolation" means dissociation of cell aggregates by an enzymatic treatment. Any enzymatic isolation method known in the art may be used without any limitation, but cell aggregates may be preferably dissociated by treatment with collagenase including collagenase I, II, III, and IV, accutase, dispase or trypsin, followed by subculturing.

Stem cells can be administered into the body by various routes, for example, intravenously, intra-arterially or intraperitoneally. Among such administration routes, intravenous administration is preferred, because it enables a disease to be treated in a convenient and safe manner without surgical operation. In order for intravenously administered stem cells to securely reach the target site and to exhibit a desired therapeutic effect, various requirements should be satisfied. First, stem cells should be administered at a certain concentration or higher concentration such that they exhibit a desired therapeutic effect after they reached the target site. Thus, it is important that stem cells which it is desired to apply clinically are obtained in large amounts. In addition, stem cells should have a size suitable for intravascular administration such that when administered intravascularly, these stem cells neither reduce blood flow velocity nor form thrombi. Moreover, stem cells should not be disrupted or aggregated before intravascular administration, and should securely reach their target site as single cells without disruption or aggregation even after intravascular administration.

In view of several requirements as described above, the present invention is intended to provide at a certain concentration or higher concentration of stem cells that are effective clinically. According to the culture of stem cells by a conventional method, a subculture must be performed many times to obtain a high yield of stem cells, and thus much manpower and time are spent. In particular, since a portion of medium components necessary for subculture is greatly expensive, it is disadvantageous in terms of economic efficiency. However, the present invention enables stem cells to be prepared in high concentration sufficient for clinical application.

In one aspect, the present invention is directed to a method for preparing a high concentration of stem cells, the method comprising a step of culturing stem cells in a medium containing a basal medium and at least two components selected from the group consisting of N-acetyl-L-cysteine (NAC), ascorbic acid, insulin or insulin-like factor, hydrocortisone, dexamethasone, bFGF (basic fibroblast growth factor), heparan sulfate, 2-mercaptoethanol, EGF (epidermal growth factor), and antioxidant.

Preferably, the stem cells used in the present invention may use adult stem cells, particularly adult stem cells obtained from adipose tissue, or epithelial tissue such as pilar cyst, amnion or the like. Most preferably, adipose tissue-derived adult stem cells may be used as the stem cells. Mesenchymal stem cells (MSCs) may be used as the stem cells, particularly adipose tissue-derived mesenchymal stem cells may be used as the stem cells. The adipose tissue or epithelial tissue is preferably derived from mammals, more preferably human. In an example of the present invention, human adipose tissue-derived mesenchymal stem cells (AdMSCs) were used.

The basal medium used in the present invention refers to a typical medium having a simple composition known as being suitable for the culture of stem cells in the art. Examples of the basal medium generally used to culture the stem cells include MEM (Minimal Essential Medium), DMEM (Dulbecco modified Eagle Medium), RPMI (Roswell Park Memorial Institute Medium), and K-SFM (Keratinocyte Serum Free Medium). As the basal medium used in the present invention, any mediums can be used without any limitation as long as they are used in the art. Preferably, the basal medium may be selected from the group consisting of M199/F12(mixture)(GIBCO), MEM-alpha medium (GIBCO), low-concentration glucose-containing DMEM medium (Welgene), MCDB 131 medium (Welgene), IMEM medium(GIBCO), K-SFM, DMEM/F12 medium, PCM medium, and MSC expansion medium (Chemicon). Particularly, among them, K-SFM may be preferably used.

A basal medium that is used to obtain the cultured mesenchymal stem cells may be supplemented with additives known in the art, which promote the proliferation of mesenchymal stem cells in an undifferentiated state while inhibiting the differentiation thereof. Also, the medium may contain a neutral buffer (such as phosphate and/or high-concentration bicarbonate) in isotonic solution, and a protein nutrient (e.g., serum such as FBS, FCS (fetal calf serum) or horse serum, serum replacement, albumin, or essential or non-essential amino acid such as glutamine or L-glutamine). Furthermore, it may contain lipids (fatty acids, cholesterol, an HDL or LDL extract of serum) and other ingredients found in most stock media of this kind (such as insulin or transferrin, nucleosides or nucleotides, pyruvate, a sugar source such as glucose, selenium in any ionized form or salt, a glucocorticoid such as hydrocortisone and/or a reducing agent such as β-mercaptoethanol).

Also, for the purpose of preventing cells from adhering to each other, adhering to a container wall, or forming too large clusters, the medium may advantageously contain an anti-clumping agent, such as one sold by Invitrogen (Cat # 0010057AE).

Among them, one or more of the following additional additives may advantageously be used:
- stem cell factor (SCF, Steel factor), other ligands or antibodies that dimerize c-kit, and other activators of the same signaling pathway
- ligands for other tyrosine kinase related receptors, such as the receptor for platelet-derived growth factor (PDGF), macrophage colony-stimulating factor, Flt-3 ligand and vascular endothelial growth factor (VEGF)
- factors that elevate cyclic AMP levels, such as forskolin
- factors that induce gp130 such as LIF or Oncostatin-M
- hematopoietic growth factors such as thrombopoietin (TPO)
- transforming growth factors such as TGFβ1
- neurotrophins such as CNTF
- antibiotics such as gentamicin, penicillin or streptomycin.

The medium that is used in the present invention may contain, in addition to the basal medium, at least two components selected from the group consisting of N-acetyl-L-cysteine (NAC), insulin or insulin-like factor, hydrocortisone, basic fibroblast growth factor (bFGF), and antioxidant.

The medium used in the present invention may contain a basal medium and at least two components selected from the group consisting of N-acetyl-L-cysteine (NAC), ascorbic acid, insulin or insulin-like factor, hydrocortisone, dexamethasone, bFGF (basic fibroblast growth factor), heparan sulfate, 2-mercaptoethanol, EGF (epidermal growth factor), and antioxidant.

Specifically, the medium may contain insulin-like factor as insulin replacement, which functions to promote cell growth by enhancing glucose metabolism and protein metabolism. Particularly, recombinant IGF-1 (insulin-like growth factor-1) is preferably used. The preferred content of insulin-like factor is 10-50 ng/ml. If the content of insulin-like factor is less than 10 ng/ml, apoptosis will occur, and if the content is more than 50 ng/ml, it will increase the cytotoxicity and cost of the medium.

The medium may contain basic fibroblast growth factor (bFGF) that can induce various types of cell proliferation *in vivo.* Preferably, recombinant bFGF protein is used. The preferred content of bFGF is 1-100 ng/ml.

Examples of an antioxidant that may be used in the present invention include selenium, ascorbic acid, vitamin E, catechin, lycopene, β-carotene, coenzyme Q-10, EPA (eicosapentaenoic acid), DHA (docosahexanoic acid) and the like. Preferably, selenium may be used. In an example of the present invention, selenium was used as an antioxidant. The content of selenium in the medium is preferably 0.5-10 ng/ml. If the content of selenium is less than 0.5 ng/ml, the medium will be sensitive to oxygen toxicity, and if the content is more than 10 ng/ml, it will cause severe cytotoxicity.

The medium that is used in the present invention may additionally contain a component selected from the group consisting of FBS (fetal bovine serum), calcium and EGF. Epidermal growth factor (EGF) can induce various types of cell proliferation *in vivo,* and recombinant EGF protein is preferably used. The preferred content of epidermal growth factor is 10-50 ng/ml. If the content of epidermal growth factor in the medium is less than 10 ng/ml, it will have no particular effect, and if the content is more than 50 ng/ml, it will be toxic to cells.

Preferably, when subcultured 3-5 times, stem cells cultured in the medium according to the present invention are proliferated at a concentration of 1 × 10⁷ - 5 × 10⁶ cells/ml. In addition, since functional/morphological deformation of cells does not occurs during the increase in the number of the stem cells by the subculture, the stem cells obtained according to the present invention can be effectively applied to a clinical trial.

In an example of the present invention, adipose-derived mesenchymal stem cells were cultured in the medium of the present invention. Adipose-derived mesenchymal stem cells can be obtained in the following manner. First, human adipose tissue obtained from the abdomen by liposuction or the like is isolated and washed with PBS, after which the tissue is cut finely and degraded using DMEM medium containing collagenase. The degraded tissue is washed with PBS and centrifuged at 1000 rpm for 5 minutes. The supernatant is removed, and the pellet remaining at the bottom is washed with PBS, and then centrifuged at 1000 rpm for 5 minutes. The resulting cells are filtered through a 100-mesh filter to remove the debris, and then washed with PBS. The cells are cultured overnight in DMEM medium (10% FBS, 2 mM NAC, 0.2 mM ascorbic acid), and then the cells that did not adhere to the bottom of the culture container were washed out with PBS, and the cells are subcultured while the medium was replaced with K-SFM medium containing NAC, ascorbic acid, calcium, rEGF, insulin, Bfgf, hydrocortisone, and selenium at 2-day intervals, thereby obtaining adipose-derived mesenchymal stem cells. In addition to this method, any method known in the art may also be used to obtain mesenchymal stem cells.

### EXAMPLES

Hereinafter, the present invention will be described in further detail with reference to examples. It will be obvious to a person having ordinary skill in the art that these examples are illustrative purposes only and are not to be construed to limit the scope of the present invention.

### Example 1: Isolation of human adipose tissue-derived mesenchymal stem cells

Adipose tissue isolated from abdominal tissue by liposuction was washed with PBS and cut finely, after which the tissue was digested in DMEM media supplemented with collagenase type 1 (1 mg/ml) at 37 °C for 2 hours. The collagenase-treated tissue was washed with PBS and centrifuged at 1000 rpm for 5 minutes. The supernatant was removed, and the pellet was washed with PBS and then centrifuged at 1000 rpm for 5 minutes. The resulting cells were filtered through a 100-mesh filter to remove debris, after which the cells were washed with PBS and cultured overnight in DMEM medium containing 10% FBS, 2 mM NAC (N-acetyl-L-cysteine) and 0.2 mM ascorbic acid.

Then, non-adherent cells were removed by washing with PBS, and the remaining cells were cultured for 4 passages while the medium was replaced with K-SFM (keratinocyte serum free medium) containing 5% FBS, 2 mM NAC, 0.2 mM ascorbic acid, 0.09 mM calcium, 5 ng/ml rEGF, 5 µg/ml insulin, 10 ng bFGF and 74 ng/ml hydrocortisone at 2-day intervals, thereby isolating adipose-derived mesenchymal stem cells.

### Example 2: Investigation of the effect of medium components on the ability of stem cells to proliferate

Medium (i.e., medium 1) containing all of FBS, N-acetyl-L-cysteine (NAC), ascorbic acid, insulin, hydrocortisone, bFGF (basic fibroblast growth factor), EGF (epidermal growth factor), and selenium, which are active ingredients added to the K-SFM medium used in Example 1 and media (i.e., media 2-9) free of at least one of the active ingredients, were prepared.

Medium composition is as follows:
Medium 1 (M1): K-SFM medium + FBS + NAC + ascorbic acid + insulin + hydrocortisone + bFGF + EGF + selenium
Medium 2 (M2): K-SFM medium + NAC + ascorbic acid + insulin + hydrocortisone + bFGF + EGF + selenium (exclusion of FBS from ingredients of medium 1)
Medium 3 (M3): K-SFM medium + FBS + NAC + ascorbic acid + insulin + hydrocortisone + EGF + selenium (exclusion of bFGF from ingredients of medium 1)
Medium 4 (M4): K-SFM medium + FBS + NAC + ascorbic acid + hydrocortisone + bFGF + EGF + selenium (exclusion of insulin from ingredients of medium 1)
Medium 5 (M5): K-SFM medium + FBS + NAC + ascorbic acid + insulin + bFGF + EGF + selenium (exclusion of hydrocortisone from ingredients of medium 1)
Medium 6 (M6): K-SFM medium + FBS + NAC + ascorbic acid + insulin + hydrocortisone + bFGF + selenium (exclusion of EGF from ingredients of medium 1)
Medium 7 (M7): K-SFM medium + FBS + NAC + insulin + hydrocortisone + bFGF + EGF + selenium (exclusion of ascorbic acid from ingredients of medium 1)
Medium 8 (M8): K-SFM medium + FBS + ascorbic acid + insulin + hydrocortisone + bFGF + EGF + selenium (exclusion of NAC from ingredients of medium 1)
Medium 9 (M9): K-SFM medium + FBS + NAC + ascorbic acid + insulin + hydrocortisone + bFGF + EGF (exclusion of selenium from ingredients of medium 1)
Medium 10 (M10): K-SFM medium + FBS + NAC + ascorbic acid + insulin + hydrocortisone + selenium (exclusion of bFGF and EGF from ingredients of medium 1).

The adipose-derived stem cells were cultured in the medium. Adipose-derived stem cells obtained from adult males aged 20, 30, 70 and 80 years were used as the adipose stem cells. After being cultured in each of the media for 4 passages, the obtained adipose-derived mesenchymal stem cells were treated with trypsin, and then the number of the cells was measured with a confocal microscope. As can be seen from Table 1 below, when 1 × 10⁵ adipose-derived mesenchymal stem cells were inoculated into the medium at 3 passages and incubated for 4 days, the number of cells obtained was listed on a basis of media and days. As a result, according to the stem cell culture method of the present invention, it could be confirmed that when the stem cells are cultured for 4 days, they could be prepared at a maximum concentration of 2.5 × 10⁶ cells/ml depending on the medium components (see Table 1)). In addition, although there is a slight difference in cell population doubling level (CPDL) by age, medium 9 showed the highest cell population doubling level (CPDL). In case of adult males aged 20 and 30 years, it could be confirmed that when the stem cells were inoculated into the medium at a concentration of 1 × 10⁵ cells/m l and incubated for 4 days, the number of the cells was increased 13-25 folds. In case of adult males aged 70 and 80 years, it could be also confirmed that the number of the cells was increased 7-15 folds (see FIG. 1). In addition, cells in which mutation occurred were not confirmed (not shown).

**Table 1: Investigation of medium composition for preparation of high concentration of stem cells**

| **Age** | **Post seeding Day** | **Total Cell Count (× 10⁴/ml)** | | | | | | | | | |
|---|---|---|---|---|---|---|---|---|---|---|---|
| | | **M1** | **M2** | **M3** | **M4** | **M5** | **M6** | **M7** | **M8** | **M9** | **M10** |
| **20** | **Day0** | 10 | 10 | 10 | 10 | 10 | 10 | 10 | 10 | 10 | 10 |
| | **Day1** | 8 | 16 | 15 | 13 | 12 | 16 | 12 | 12 | 8 | 12 |
| | **Day2** | 39 | 13.5 | 30.5 | 38 | 29.5 | 33.5 | 39 | 30 | 26.5 | 9.5 |
| | **Day3** | 115 | 11.5 | 70 | 110 | 115 | 110 | 110 | 110 | 105 | 11 |
| | **Day4** | 230 | 10.5 | 135 | 195 | 180 | 225 | 250 | 230 | 205 | 13 |
| **30** | **Day0** | 10 | 10 | 10 | 10 | 10 | 10 | 10 | 10 | 10 | 10 |
| | **Day1** | 12 | 10 | 14 | 11 | 16 | 27 | 23 | 10 | 26 | 25 |
| | **Day2** | 35 | 12 | 24.5 | 40 | 40 | 35.5 | 33.5 | 39.5 | 41 | 10.5 |
| | **Day3** | 120 | 150 | 0 | 90 | 110 | 95 | 105 | 110 | 125 | 10.5 |
| | **Day4** | 215 | 10 | 150 | 185 | 130 | 165 | 230 | 220 | 220 | 13 |
| **70** | **Day0** | 10 | 10 | 10 | 10 | 10 | 10 | 10 | 10 | 10 | 10 |
| | **Day1** | 12 | 7 | 16 | 12 | 13 | 20 | 10 | 13 | 20 | 11 |
| | **Day2** | 37 | 9 | 31 | 32.5 | 27.5 | 29 | 29.5 | 30.5 | 26 | 13 |
| | **Day3** | 85 | 12 | 60 | 65 | 80 | 86 | 90 | 100 | 95 | 12 |
| | **Day4** | 155 | 7 | 85 | 120 | 135 | 105 | 145 | 150 | 155 | 13.5 |
| **80** | **Day0** | 10 | 10 | 10 | 10 | 10 | 10 | 10 | 10 | 10 | 10 |
| | **Day1** | 20 | 10 | 17 | 15 | 16 | 13 | 16 | 13 | 17 | 16 |
| | **Day2** | 24.5 | 11.5 | 16.5 | 21 | 19.5 | 26 | 17 | 24.5 | 23 | 10.5 |
| | **Day3** | 37 | 7.5 | 31 | 41 | 37 | 47 | 65 | 55 | 48.5 | 9 |
| | **Day4** | 75 | 9 | 55 | 90 | 70 | 70 | 65 | 80 | 65 | 14.5 |

### INDUSTRIAL APPLICABILITY

According to the present invention, since stem cells can be prepared in an amount sufficient for clinical application even in culture for 3-5 passages, the effect of intravascular administration of the stem cells on cell therapy can be significantly increased.

Although the present invention has been described in detail with reference to the specific features, it will be apparent to those skilled in the art that this description is only for a preferred embodiment and does not limit the scope of the present invention. Thus, the substantial scope of the present invention will be defined by the appended claims and equivalents thereof.

## Claims

1. A method for preparing stem cells at a high concentration of 1 × 10⁷ - 5 × 10⁸ cells/ml, the method comprises of culturing stem cells in a medium containing a basal medium; and at least two components selected from the group consisting of N-acetyl-L-cysteine (NAC), ascorbic acid, insulin or insulin-like factor, hydrocortisone, dexamethasone, bFGF (basic fibroblast growth factor), heparan sulfate, 2-mercaptoethanol, EGF (epidermal growth factor), and antioxidant.

2. The method of claim 1, wherein the basal medium is selected from the group consisting of M199/F12 (mixture)(GIBCO), MEM-alpha medium (GIBCO), low-concentration glucose-containing DMEM medium (Welgene), MCDB 131 medium (Welgene), IMEM medium(GIBCO), K-SFM, DMEM/F12 medium, PCM medium, and MSC expansion medium (Chemicon).

3. The method of claim 1, wherein the antioxidant is selected from the group consisting of selenium, ascorbic acid, vitamin E, catechin, lycopene, β-carotene, coenzyme Q-10, EPA (eicosapentaenoic acid), and DHA (docosahexanoic acid).

4. The method of claim 3, wherein the antioxidant is selenium.

5. The method of claim 1, wherein the medium additionally contain a component selected from the group consisting of FBS (fetal bovine serum), calcium, and EGF.

6. The method of claim 1, wherein the stem cells are adipose tissue-derived mesenchymal stem cells.
